(19) Europäisches Patentamt
European Patent Office
Office européen des brevets

(11) Veröffentlichungsnummer: **0 129 500**
**B1**

(12) # EUROPÄISCHE PATENTSCHRIFT

(45) Veröffentlichungstag der Patentschrift: **05.08.87**

(51) Int. Cl.⁴: **C 07 J 1/00**, A 61 K 31/565

(21) Anmeldenummer: **84730063.9**

(22) Anmeldetag: **13.06.84**

(54) **1-Alkyl-androsta-1,4-dien-3,17-dione, Verfahren zu deren Herstellung und diese enthaltende pharmazeutische Präparate.**

(30) Priorität: **18.06.83 DE 3322285**

(43) Veröffentlichungstag der Anmeldung: **27.12.84 Patentblatt 84/52**

(45) Bekanntmachung des Hinweises auf die Patenterteilung: **05.08.87 Patentblatt 87/32**

(84) Benannte Vertragsstaaten: **AT BE CH DE FR GB IT LI LU NL SE**

(56) Entgegenhaltungen:
**DE-B-1 174 776**
**DE-B-1 174 778**
**DE-B-1 249 268**
**US-A-3 006 929**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Kerb, Ulrich, Dr., Prinzregentenstrasse 7, D-1000 Berlin 31 (DE)**
Erfinder: **Sauer, Gerhard, Dr., Königsbacher Zeile 47 A, D-1000 Berlin 28 (DE)**
Erfinder: **Wiechert, Rudolf, Prof. Dr., Petzower Strasse 8 A, D-1000 Berlin 39 (DE)**
Erfinder: **Henderson, David, Dr., Jahnstrasse 17, D-1000 Berlin 28 (DE)**
Erfinder: **Nishino, Yukishige, Dr., Lanzendorfer Weg 14, D-1000 Berlin 22 (DE)**
Erfinder: **Beier, Sybille, Dr., Uhlandstrasse 121, D-1000 Berlin 31 (DE)**

**EP 0 129 500 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 1-Alkyl-androsta-1,4-dien-3,17-dione der allgemeinen Formel I, Verfahren zu deren Herstellung und diese enthaltende pharmazeutische Präprate.

Die neuen 1-Alkyl-androsta-1,4-dien-3,17-dione werden durch die allgemeine Formel I gekennzeichnet

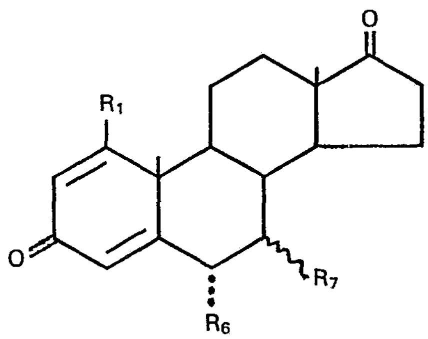
(I),

worin

$R_1$ eine Methyl- Ethyl-, Hydroxymethyl-, $C_1$-$C_3$-Alkoxymethyl- oder $C_1$-$C_4$-Alkanoyloxymethyl-Gruppe,

$R_6$ ein Wasserstoffatom oder eine Methylgruppe und

$R_7$ ein Wasserstoffatom oder eine Methylgruppe in 7α- oder 7β-Stellung

bedeuten.

Es wurde gefunden, dass die neuen Verbindungen der allgemeinen Formel I die Östrogenbiosynthese hemmen. Das ist überraschend; denn die entsprechenden in 1-Stellung unsubstituierten Androsta-1,4-dien-3,17-dione lassen den Östrogenspiegel deutlich ansteigen.

Bei der Biosynthese von Östrogenen spielt die Umwandlung von Androgenen zu Östrogenen eine wichtige Rolle. Diese Umwandlung verläuft über eine Reihe von Reaktionen, die zusammengefasst als «Aromatisierung» bezeichnet werden. Das Enzym Aromatase ist das geschwindigkeitsbestimmende Enzym bei der Umwandlung von Androstendion und Testosteron zu Östron und Östradiol.

Die erfindungsgemässen Verbindungen wurden auf Aromatasehemmwirkung und auf endokrinpharmakologische Nebenwirkungen untersucht.

A: 4-Hydroxy-4-androsten-3,17-dion (als Vergleichssubstanz der Prüfung auf Aromatasehemmwirkung, auf östrogene Wirkung und im Hodenhemmtest).

B: 1-Methyl-androsta-1,4-dien-3,17-dion.

C: 1,7α-Dimethyl-androsta-1,4-dien-3,17-dion.

D: 1,6α-Dimethyl-androsta-1,4-dien-3,17-dion.

1.) *PMSG-Test bei der Ratte zur Prüfung auf Aromatasehemmwirkung*

Es wird die Beeinflussung der PMSG (Pregnant Mare Serum Gonadotropin)-stimulierten Östrogenproduktion durch die Prüfsubstanzen A, B, C und D untersucht. Die durch Behandlung mit PMSG vermehrte Sekretion von Östradiol (Kontrolle) kann durch Applikation von Aromatasehemmern vermindert werden.

20 Tage alte weibliche Ratten wurden alle 2 Tage insgesamt 7 × mit 100 I.U. PMSG subcutan vorbehandelt. Eine Stunde vor und 8 Stunden nach der letzten PMSG-Applikation ($d_{12}$) erhielten die Tiere die Testsubstanz durch Injektion. Die Kontrolltiere erhielten nur das Vehikel. 24 Stunden nach der letzten PMSG-Applikation wurden die Tiere getötet. Im Serum wurde dann Östradiol radioimmunologisch bestimmt. Angegeben werden die Östradiolkonzentration in nMol/l und die Standardabweichung.

Die Signifikanzen der Unterschiede zur Kontrollgruppe wurden durch die Varianzanalyse geprüft. Die Wirkungsweise wurde durch die Regressions-/Kovarianzanalyse ermittelt.

TABELLE 1

Beeinflussung der Östradiolkonzentration im peripheren Serum bei der PMSG-vorbehandelten Ratte

| | Dosis mg/Tier 2 × s.c. | n | Östradiol-Konzentration nMol/L | Relative Wirkungsstärke (A = 1,0) |
|---|---|---|---|---|
| PMSG-Kontrolle | (0,2 ml Vehikel | 10 | 4,25 ± 1,57 | |
| A | 0,1 | 10 | 4,31 ± 1,78 | 1,0 |
| | 0,3 | 10 | 3,19 ± 1,31 | |
| | 1,0 | 10 | 1,58 ± 0,92* | |
| | 3,0 | 10 | 1,52 ± 0,74* | |
| B | 0,1 | 10 | 3,53 ± 1,84 | 1,3 |
| | 0,3 | 10 | 2,92 ± 1,09 | |
| | 1,5 | 10 | 1,80 ± 1,13* | |
| | 3,0 | 10 | 0,78 ± 0,18* | |

TABELLE 1 (Fortsetzung)

| | Dosis mg/Tier 2 × s.c. | n | Östradiol-Konzentration nMol/L | Relative Wirkungsstärke (A = 1,0) |
|---|---|---|---|---|
| C | 0,1 | 10 | 2,21 ± 1,05* | 6,3 |
| | 0,3 | 10 | 0,81 ± 0,32* | |
| | 1,0 | 10 | 0,49 ± 0,21* | |
| | 3,0 | 10 | 0,37 ± 0,11* | |
| D | 0,1 | 10 | 3,73 ± 0,89 | 1,2 |
| | 0,3 | 10 | 2,78 ± 1,17 | |
| | 1,0 | 10 | 1,78 ± 0,67* | |
| | 3,0 | 10 | 1,02 ± 0,38* | |

* = signifikanter Unterschied gegen die PMSG-Kontrolle
n = Tierzahl pro Gruppe

Die erfindungsgemässen Verbindungen B, C und D bewirken — ähnlich wie die Vergleichssubstanz A — eine dosisabhängige Abnahme der durch PMSG erhöhten Östradiolkonzentration im peripheren Serum bei der Ratte (Tabelle 1). B und D sind dabei etwa gleich stark wirksam wie die Vergleichssubstanz A, während C etwa 6mal wirksamer ist als die Vergleichssubstanz A.

2.) *Uterus/Vagina-Wachstumstest bei Mäusen und Ratten zur Prüfung auf östrogene Wirkung*

Ovariektomierte Ratten (200 g) bzw. Mäuse (30 g) erhielten täglich einmal 5 Tage lang die Testsubstanz subcutan. Die Kontrolltiere erhielten nur das Vehikel.

Einen Tag nach der letzten Behandlung wurden die Tiere getötet. Der Uterus und die Vagina wurden dann sofort herauspräpariert und gewogen.

Für jede Gruppe wurde der Mittelwert des Organgewichts und die Standardabweichung ermittelt. Die Signifikanzen der Unterschiede zur Kontrolle wurden durch die Varianzanalyse geprüft.

TABELLE 2

Östrogentest bei ovariektomierten Mäusen

| | Dosis mg/Tier/d s.c. | Organgewicht [mg] Uterus | Vagina |
|---|---|---|---|
| $E_2$-Kontrolle | 0.00003 | 126,5 ± 20,5* | 55,9 ± 7,8 |
| A | 3 | 48,4 ± 8,8* | 16,6 ± 3,3 |
| | 10 | 64,7 ± 13,1* | 23,1 ± 4,4 |
| B | 3 | 23,8 ± 6,7 | 17,7 ± 4,5 |
| | 10 | 13,7 ± 3,0 | 10,6 ± 1,5 |
| Öl-Kontrolle | — | 32,3 ± 9,2 | 23,5 ± 6,5 |

* = signifikanter Unterschied gegen die Kontrolle
$E_2$ = Estradiol

TABELLE 3

Östrogentest bei ovariektomierten Ratten

| | Dosis mg/Tier/d s.c. | Organgewicht [mg] Uterus | Vagina |
|---|---|---|---|
| $E_2$-Kontrolle | 0.0001 | 214,4 ± 25,1* | 102,3 ± 11,8* |
| A | 3 | 94,3 ± 21,9 | 52,9 ± 5,7 |
| | 10 | 131,9 ± 21,0 | 68,1 ± 6,2 |

TABELLE 3 (Fortsetzung)

| | Dosis mg/Tier/d s.c. | Organgewicht [mg] | |
|---|---|---|---|
| | | Uterus | Vagina |
| B | 3 | 85,0 ± 7,6 | 55,8 ± 7,4 |
| | 10 | 87,4 ± 21,1 | 56,6 ± 9,4 |
| Öl-Kontrolle | — | 94,5 ± 22,8 | 66,0 ± 20,5 |

* = signifikanter Unterschied gegen die Kontrolle
$E_2$ = Estradiol

Die erfindungsgemässe Verbindung B (3 und 10 mg/Tier/Tag, 5 × s.c.) zeigt weder bei der Ratte noch bei der Maus eine östrogene Wirkung (Tabellen 2 und 3). Die Vergleichssubstanz A führt dagegen bei einer Dosis von 3 und 10 mg/Tier/Tag (5 × s.c.) zu einer signifikanten Zunahme der Uterusgewichte.

3.) *Hodenhemmtest bei der Ratte zur Prüfung auf antigonadotrope Wirkung*

Männliche infantile Ratten (30 g) wurden täglich einmal 12 Tage lang mit der Testsubstanz subcutan behandelt. Einen Tag nach der letzten Behandlung wurden die Tiere getötet und die Hodengewichte ermittelt. Die Kontrolltiere erhielten nur das Vehikel.

Zur Kontrolle wurden die Organgewichte der Prostata, Samenblasen und Nebennieren festgestellt.

Die Organgewichte wurden auf mg/100 g Körpergewicht umgerechnet. Für jede Gruppe wurden der Mittelwert und die Standardabweichung errechnet.

Die Signifikanzen der Unterschiede zur Kontrolle wurden durch die Varianzanalyse geprüft.

TABELLE 4

Hodenhemmtest bei der Ratte

| | Dosis mg/Tier/Tag | n | Organgewichte mg/100 g Körpergewicht | | | |
|---|---|---|---|---|---|---|
| | | | Hoden | Samenblase | Prostata | Nebenniere |
| B | 1 | 10 | 891 ± 75 | 12,8 ± 4,4* | 40,1 ± 6,7 | 32,4 ± 5,4 |
| | 3 | 10 | 913 ± 70 | 14,5 ± 3,6 | 42,0 ± 7,7 | 27,4 ± 3,4 |
| A | 1 | 10 | 779 ± 125* | 9,6 ± 2,6* | 43,3 ± 9,8 | 23,9 ± 2,9* |
| | 3 | 10 | 633 ± 50* | 14,4 ± 3,0 | 57,5 ± 11,7 | 26,1 ± 4,3 |
| Kontrolle (Öl) | — | 10 | 1012 ± 129 | 17,8 ± 5,1 | 51,3 ± 17,0 | 30,3 ± 3,1 |

n = Tierzahl pro Gruppe
* = signifikanter Unterschied gegen die Kontrolle

Die erfindungsgemässe Verbindung B hat bei den getesteten Dosen (1 und 3 mg/Tier/Tag 12 × s.c.) im Hodenhemmtest keinen Einfluss auf die Hodengewichte (keine antigonadotrope Wirkung), bewirkt bei der Dosis von 1 mg/Tier/Tag aber eine leichte Abnahme der Samenblasengewichte. Dagegen führt die Vergleichssubatanz A zu einer signifikanten Abnahme der Organgewichte der Hoden, Samenblasen und Nebennieren.

Im Anabol/Androgen-Test hat die erfindungsgemässe Verbindung B keinen Einfluss auf die Frischgewichte von Samenblasen, Prostata, M. levator ani und Nebennieren.

Aus den Versuchen 1 bis 3 ergibt sich, dass die erfindungsgemässen Verbindungen starke Aromataseinhibitoren darstellen, die endokrinpharmakologisch weitgehend neutral sind.

Als Aromataseinhibitoren sind die neuen Verbindungen der allgemeinen Formel I zur Hemmung der Östrogenbiosynthese und damit zur Behandlung von Krankheiten geeignet, die durch Östrogene bedingt oder von Östrogenen abhängig sind.

Einen Östrogenüberschuss findet man häufig bei Frauen in der Menopause mit einem Risiko, an Brustkrebs zu erkranken.

Bei Männern führt eine vermehrte Östrogenproduktion und ein erhöhtes Östrogen/Androgen-Verhältnis zur Gynäkomastie und zur Prostatahyperplasie.

Die Verbindungen eignen sich somit zur Behandlung von Brustkrebs und anderer östrogeninduzierter oder -stimulierter Tumoren.

Aromataseinhibitoren sind auch vorteilhaft geeignet zur prophylaktischen und/oder therapeutischen Behandlung der Prostatahyperplasie.

Die neuen Verbindungen sind auch wertvoll zur Beeinflussung der Fertilität, beispielsweise zur Behandlung der männlichen Infertilität, die aus erhöhten

Östrogenspiegeln resultiert. Ferner können die Verbindungen als Antifertilitätsmittel verwendet werden, um Ovulation oder Implantation bei Frauen im fortpflanzungsfähigen Alter zu verhindern.

Die Verbindungen können oral oder parenteral, beispielsweise intraperitoneal, intramuskulär, subkutan oder perkutan, verabreicht werden. Die Verbindungen können auch in das Gewebe implantiert werden.

Die zu verabreichende Menge der Verbindung schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg/kg Körpergewicht, vorzugsweise 0,1 - 20 mg/kg Körpergewicht, je Tag betragen.

Zur oralen Verabreichung kommen Kapseln, Pillen, Tabletten, Dragées usw. in Frage. Die Dosierungseinheiten können neben dem Wirkstoff einen pharmazeutisch verträglichen Träger, wie z.B. Stärke, Zucker, Sorbit, Gelatine, Gleitmittel, Kieselsäure, Talkum usw., enthalten. Die einzelnen Dosierungseinheiten für die orale Applikation können beispielsweise 10 bis 100 mg des Wirkstoffs (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind z.B. Olivenöl, Erdnussöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, dass eine verzögerte Wirkstoff-Freigabe ermöglicht wird.

Implantate können als inerte Materialien z.B. biologisch abbaubare Polymere enthalten oder synthetische Silikone wie z.B. Silikonkautschuk. Die Wirkstoffe können ausserdem zur perkutanen Applikation z.B. in ein Pflaster eingearbeitet werden.

Die Erfindung betrifft somit auch pharmazeutische Präparate, die Verbindungen der allgemeinen Formel I enthalten.

Die neuen Verbindungen der allgemeinen Formel I werden erfindungsgemäss dadurch hergestellt, dass man

a) in einem 17-Hydroxysteroid der allgemeinen Formel II

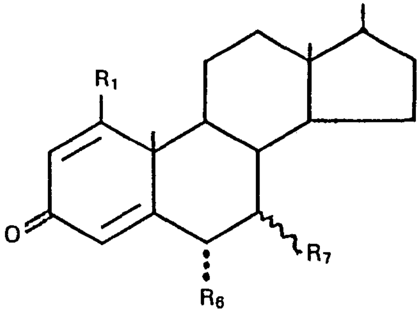

(II),

worin $R_1$, $R_6$ und $R_7$ die in Formel I angegebene Bedeutung haben, die 17-Hydroxygruppe zur 17-Oxogruppe oxydiert oder

b) ein im A-Ring gesättigtes oder einfach ungesättigtes Steroid der allgemeinen Formel III

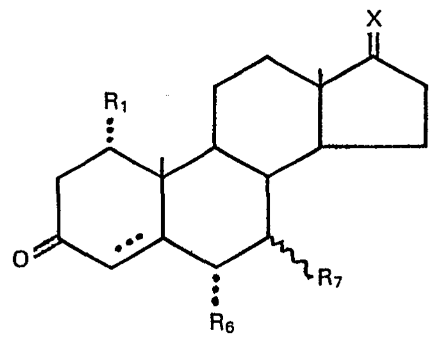

(III),

worin X O, H(OH) oder H(OAc) bedeutet,
wobei Ac für eine niedere 1-4 C-Atome enthaltende Acylgruppe steht,
$R_1$, $R_6$ und $R_7$ die in Formel I angegebene Bedeutung haben und die $\Delta^4$-Doppelbindung fakultativ ist, in 1,2- oder 1,2- und 4,5-Stellung dehydriert und eine freie oder freigesetzte 17-Hydroxygruppe oxydiert oder

c) ein im A-Ring gesättigtes Steroid der allgemeinen Formel IV

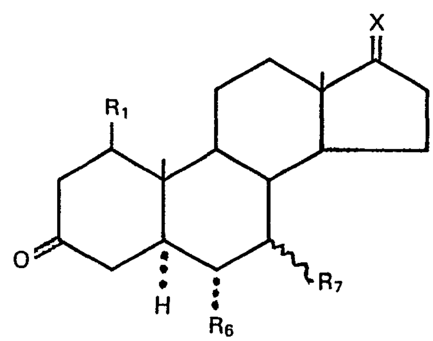

(IV),

worin X O, H(OH) oder H(OAc) bedeutet,
wobei Ac für eine niedere 1-4 C-Atome enthaltende Acylgruppe steht und
$R_1$, $R_6$ und $R_7$ die in Formel I angegebene Bedeutung haben, in 1,2- und 4,5-Stellung dehydriert und eine freie oder freigesetzte 17-Hydroxygruppe oxydiert oder

d) die Alkanoyloxygruppe in einem 1-Alkanoyloxymethylsteroid der allgemeinen Formel I'

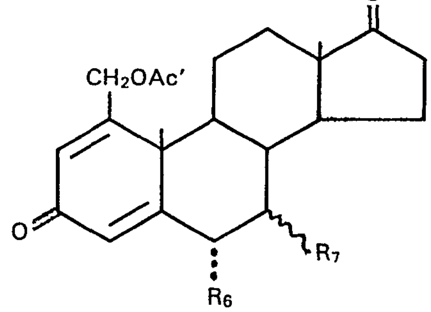

(I'),

worin Ac′ eine Alkanoylgruppe mit 1 bis 4 Kohlenstoffatomen darstellt und $R_6$ und $R_7$ die in Formel I angegebene Bedeutung haben, verseift und gegebenenfalls die freigesetzte Hydroxygruppe im 1-Hydroxymethylsteroid in einen $C_{1-3}$-Alkylether oder einen von OAc′ verschiedenen $C_{1-4}$-Alkanoylester überführt oder

e) ein Hydroxymethylsteroid der allgemeinen Formel I mit p-Toluolsulfonsäurechlorid und Pyridin zum 1-Tosylmethylsteroid und anschliessend mit Alkalialkoholat zum Alkoxymethylsteroid der allgemeinen Formel I umsetzt.

Die Umsetzungen a) bis e) erfolgen nach bekannten Methoden der Steroidchemie.

Die Oxydation gemäss Verfahrensvariante a) kann in an sich bekannter Weise, z.B. mit Chromsäurereagenzien (Jones′-Reagenz oder Chromsäure-Pyridin) oder mit Pyridiniumdichromat oder -chlorchromat durchgeführt werden.

Die 1,2-Dehydrierung gemäss Verfahren b) kann nach bekannten Methoden mit Dehydrierungsmitteln, wie Selendioxid oder Dichlordicyanobenzochinon, vorgenommen werden.

Doppelbindungen in 1,2- und 4,5-Stellung können gleichzeitig eingeführt werden, z.B. durch Bromierung zum 2,4-Dibrom-3-Keton und Dehydrobromierung des Dibromids mit Lithium- oder Calciumcarbonat und Lithiumbromid in Dimethylformamid.

Eine bei der Umsetzung freigesetzte 1-Hydroxymethylgruppe kann anschliessend verestert oder verethert werden, und eine freigesetzte 17-Hydroxygruppe kann anschliessend oxydiert werden.

Die Veresterung der 1-Hydroxymethylgruppe in Gegenwart einer 17-Hydroxygruppe erfolgt vorzugsweise mit Bleidiacetat und Acetanhydrid in Dimethylformamid.

Die Oxydation der 17β-Hydroxygruppe wird nach Verfahrensvariante a) durchgeführt.

Für die Dehydrierung gemäss Verfahrensvariante c) kommen sowohl die chemischen Dehydrierungsmittel als auch die mikrobiologische Dehydrierung in Frage. Geeignete Mikroorganismen zur Einführung der 1,2- und 4,5-Doppelbindung sind z.B. Schizomyceten, wie z.B. Arthrobacter simplex (ATCC 6946), Bacillus lentus (ATCC 13805) oder Bacillus sphaericus (ATCC 7055).

Die Verseifung einer 1-Alkanoyloxymethylgruppe gemäss Verfahren d) kann mit einer anorganischen Base in alkoholischer Lösung erfolgen; die sich gegebenenfalls anschliessende erneute Veresterung wird vorzugsweise mit dem entsprechenden Säureanhydrid in Gegenwart von organischen Basen vorgenommen.

Zur Überführung des 1-Hydroxymethylsteroids der allgemeinen Formel I in den entsprechenden 1-Alkoxymethylether wird nach einer bevorzugten Ausführungsform das 1-Hydroxymethylsteroid mit p-Toluolsulfonsäurechlorid und Pyridin zum 1-Tosyloxymethylsteroid und anschliessend mit Alkalialkoholat zum 1-Alkoxymethylsteroid umgesetzt.

Die folgenden Beispiele sollen das erfindungsgemässe Verfahren näher erläutern:

*Beispiel 1*

6,01 g 17β-Hydroxy-1-methyl-androsta-1,4-dien-3-on werden in 100 ml Aceton gelöst. Zu dieser Lösung werden 22 ml einer Chromsäurelösung (hergestellt aus 6,67 g $CrO_3$, 6 ml konzentrierter $H_2SO_4$ und Auffüllen mit Wasser auf 100 ml) bei Raumtemperatur getropft. Es wird 1 Stunde nachgerührt und anschliessend in Eiswasser gefällt, das Produkt wird abgesaugt und getrocknet. Man erhält nach Umkristallisieren aus Aceton/Hexan 5,25 g 1-Methyl-androsta-1,4-dien-3,17-dion vom Schmelzpunkt 165 bis 166°C.

*Beispiel 2*

30 g 1α,7α-Dimethyl-4-androsten-3,17-dion werden in 700 ml Dioxan mit 30 g Dichlordicyanobenzochinon 8 Stunden unter Rückfluss erhitzt. Nach Abkühlen wird vom Ungelösten abgesaugt, mit Dioxan gewaschen und das Filtrat im Vakuum eingeengt. Der Rückstand wird an Silikagel chromatographiert und aus Aceton/Hexan umkristallisiert. Man erhält 18 g 1,7α-Dimethyl-androsta-1,4-dien-3,17-dion vom Schmelzpunkt 148-151°C.

Analog werden hergestellt:

1,7β-Dimethyl-androsta-1,4-dien-3,17-dion vom Schmelzpunkt 196-197°C aus 1α,7β-Dimethyl-4-androsten-3,17-dion,

1,6α-Dimethyl-androsta-1,4-dien-3,17-dion vom Schmelzpunkt 234-235°C aus 1α,6α-Dimethyl-4-androsten-3,17-dion,

1-Ethyl-androsta-1,4-dien-3,17-dion vom Schmelzpunkt 175-176°C aus 1α-Ethyl-4-androsten-3,17-dion.

*Beispiel 3*

9,1 g 1α-Acetoxymethyl-17β-acetoxy-5α-androstan-3-on (Schmelzpunkt 193-194°C) [hergestellt aus 3,3-Ethylendioxy-1-methylen-5α-androstan-17β-ol (Naturwissenschaften 51, 86 (1963)), durch Hydroborierung, Acetylierung und Ketalspaltung] werden in 90 ml Eisessig gelöst. Zu dieser Lösung werden 3,2 ml Brom in 25 ml Eisessig getropft, und es wird 10 Minuten nachgerührt. Nach Fällung in natriumsulfithaltigem Eiswasser wird das Produkt abgesaugt, mit Wasser gewaschen und getrocknet. 16,5 g rohes Dibromid werden in 100 ml Dimethylformamid mit 10 g Lithiumbromid und 20 g Calciumcarbonat 3,5 Stunden bei 120°C Badtemperatur gerührt. Nach dem Abkühlen werden die anorganischen Salze abgesaugt und mit 150 ml Methanol nachgewaschen. Das Filtrat wird mit 6 g Kaliumhydroxid in 20 ml Wasser versetzt und 16 Stunden bei Raumtemperatur gerührt. Die Reaktionslösung wird in Eiswasser eingegossen, das Produkt abgesaugt und getrocknet. Das rohe 1-Hydroxymethyl-17β-hydroxy-androstra-1,4-dien-3-on (7,5 g) wird in 100 ml Dimethylformamid gelöst, mit 1 g Bleidiacetat und 15 ml Acetanhydrid versetzt und 6,5 Stunden bei Raumtemperatur gerührt. Nach Fällung in Wasser und Absaugen des Produktes wird wie in Beispiel 1 beschrieben mit Chromsäure oxidiert. Man erhält nach Umkristallisation aus Aceton/Hexan 2,3 g 1-Acetomethyl-androsta-1,4-dien-3,17-dion vom Schmelzpunkt 121-123°C.

*Beispiel 4*

2,2 g 1-Acetoxymethyl-androsta-1,4-dien-3,17--dion werden in 20 ml Methylenchlorid und 25 ml Methanol mit 250 mg Kaliumhydroxid bei 0-5°C unter Argonbegasung 5 Stundenn gerührt. Anschliessend wird mit Essigsäure neutralisiert, eingeengt und in Wasser gefällt. Das Produkt wird abgesaugt, mit Wasser gewaschen und getrocknet. Nach Umkristallisation aus Aceton/Hexan erhält man 1,7 g 1-Hydroxymethyl-androsta-1,4-dien-3,17-dion vom Schmelzpunkt 201-202°C.

*Beispiel 5*

500 mg 1-Hydroxymethyl-androsta-1,4-dien--3,17-dion werden in 5 ml Pyridin gelöst, auf 0°C gekühlt, mit 350 mg p-Toluolsulfonylchlorid versetzt und 3 Stunden bei Raumtemperatur gerührt. Nach Wasserfällung des Produkts, Absaugen, Waschen mit Wasser und Trocknen werden 700 mg rohes Tosylat erhalten. Dieses Rohprodukt wird in 5 ml Toluol mit 500 mg Kaliumethylat 5 Stunden bei 20°C gerührt. Anschliessend wird mit Ether verdünnt, mit verdünnter Salzsäure und mit Wasser gewaschen und im Vakuum eingeengt. Nach Chromatographie an Silicagel und Umkristallisation aus Hexan/Aceton erhält man 325 mg 1-Ethoxymethyl-androsta-1,4--dien-3,17-dion vom Schmelzpunkt 95-98°C.

**Patentansprüche für die Vertragsstaten:**
BE, CH, DE, FR, GB, IT, LI, LU, NL, SE

1. 1-Alkyl-androsta-1,4-dien-3,17-dione der allgemeinen Formel I

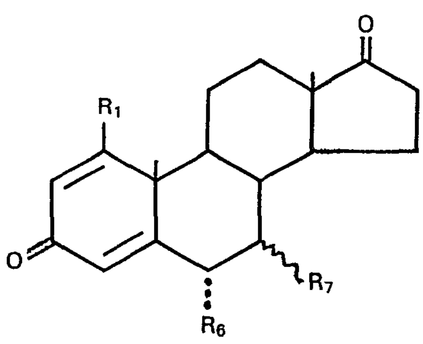

(I),

worin
$R_1$ eine Methyl-, Ethyl-, Hydroxymethyl-, $C_1$-$C_3$--Alkoxymethyl- oder $C_1$-$C_4$-Alkanoyloxymethyl-Gruppe,
$R_6$ ein Wasserstoffatom oder eine Methylgruppe und
$R_7$ ein Wasserstoffatom oder eine Methylgruppe in 7α- oder 7β-Stellung
bedeuten.

2. Methyl-androsta-1,4-dien-3,17-dion.

3. 1,7α-Dimethyl-androsta-1,4-dien-3,17-dion und 1,7β-Dimethyl-androsta-1,4-dien-3,17-dion.

4. 1,6α-Dimethyl-androsta-1,4-dien-3,17-dion.

5. 1-Ethyl-androsta-1,4-dien-3,17-dion.

6. 1-Acetoxymethyl-androsta-1,4-dien-3,17--dion.

7. 1-Hydroxymethyl-androsta-1,4-dien-3,17--dion.

8. 1-Ethoxymethyl-androsta-1,4-dien-3,17--dion.

9. Pharmazeutische Präparate, gekennzeichnet durch den Gehalt an einer Verbindung gemäss Anspruch 1 bis 8.

10. Verfahren zur Herstellung von 1-Alkyl-androsta-1,4-dien-3,17-dionen der allgemeinen Formel I

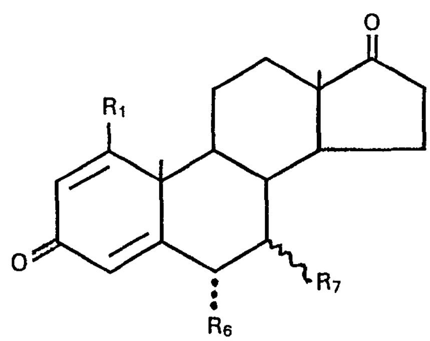

(I),

worin
$R_1$ eine Methyl-, Ethyl-, Hydroxymethyl-, $C_1$-$C_3$--Alkoxymethyl- oder $C_1$-$C_4$-Alkanoyloxymethyl-Gruppe,
$R_6$ ein Wasserstoffatom oder eine Methylgruppe und
$R_7$ ein Wasserstoffatom oder eine Methylgruppe in 7α- oder 7β-Stellung
bedeuten,
dadurch gekennzeichnet, dass man
a) in einem 17-Hydroxysteroid der allgemeinen Formel II

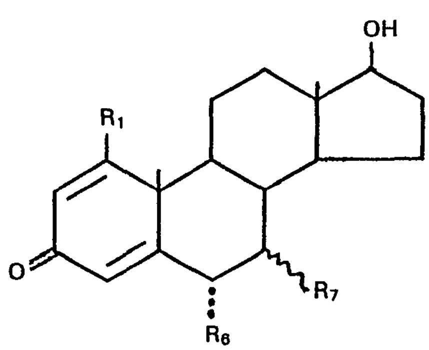

(II),

worin $R_1$, $R_6$ und $R_7$ die in Formel I angegebene Bedeutung haben, die 17-Hydroxygruppe zur 17-Oxogruppe oxydiert oder
b) ein im A-Ring gesättigtes oder einfach ungesättigtes Steroid der allgemeinen Formel III

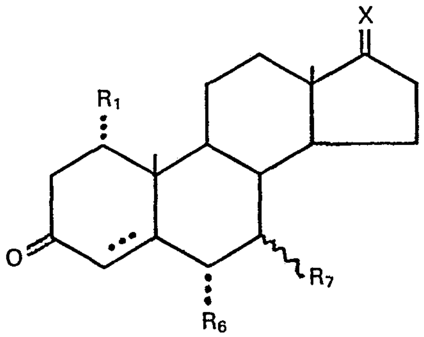

(III),

worin X O, H(OH) oder H(OAc) bedeutet, wobei Ac für eine niedere 1-4 C-Atome enthaltende Acylgruppe steht, $R_1$, $R_6$ und $R_7$ die in Formel I angegebene Bedeutung haben und die $\Delta^4$-Doppelbindung fakultativ ist, in 1,2- oder 1,2- und 4,5-Stellung dehydriert und eine freie oder freigesetzte 17-Hydroxygruppe oxydiert oder

c) ein im A-Ring gesättigtes Steroid der allgemeinen Formel IV

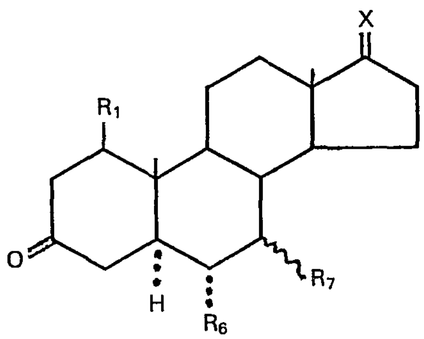

(IV),

worin X O, H(OH) oder H(OAc) bedeutet, wobei Ac für eine niedere 1-4 C-Atome enthaltende Acylgruppe steht und $R_1$, $R_6$ und $R_7$ die in Formel I angegebene Bedeutung haben, in 1,2- und 4,5-Stellung dehydriert und eine freie oder freigesetzte 17-Hydroxygruppe oxydiert oder

d) die Alkanoyloxygruppe in einem 1-Alkanoyloxymethylsteroid der allgemeinen Formel I'

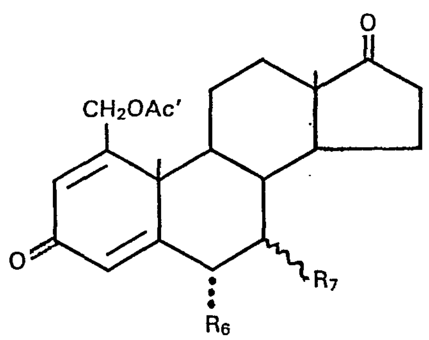

(I'),

worin Ac' eine Alkanoylgruppe mit 1 bis 4 Kohlenstoffatomen darstellt und $R_6$ und $R_7$ die in Formel I angegebene Bedeutung haben, verseift und gegebenenfalls die freigesetzte Hydroxygruppe im 1-Hydroxymethylsteroid in einen $C_{1-3}$-Alkylether oder einen von OAc' verschiedenen $C_{1-4}$-Alkanoylester überführt oder

e) ein Hydroxymethylsteroid der allgemeinen Formel I mit p-Toluolsulfonsäurechlorid und Pyridin zum 1-Tosylmethylsteroid und anschliessend mit Alkalialkoholat zum Alkoxymethylsteroid der allgemeinen Formel I umsetzt.

**Patentanspruch für den Vertragsstaat:** AT

Verfahren zur Herstellung von 1-Alkyl-androsta-1,4-dien-3,17-dionen der allgemeinen Formel I

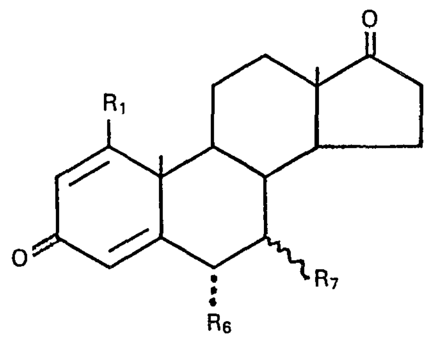

(I),

worin

$R_1$ eine Methyl-, Ethyl-, Hydroxymethyl-, $C_1$-$C_3$-Alkoxymethyl- oder $C_1$-$C_4$-Alkanoyloxymethyl-Gruppe,

$R_6$ ein Wasserstoffatom oder eine Methylgruppe und

$R_7$ ein Wasserstoffatom oder eine Methylgruppe in 7α- oder 7β-Stellung

bedeuten,

dadurch gekennzeichnet, dass man

a) in einem 17-Hydroxysteroid der allgemeinen Formel II

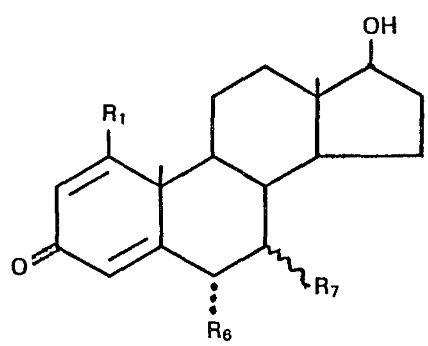

(II),

worin $R_1$, $R_6$ und $R_7$ die in Formel I angegebene Bedeutung haben, die 17-Hydroxygruppe zur 17-Oxogruppe oxydiert oder

b) ein im A-Ring gesättigtes oder einfach ungesättigtes Steroid der allgemeinen Formel III

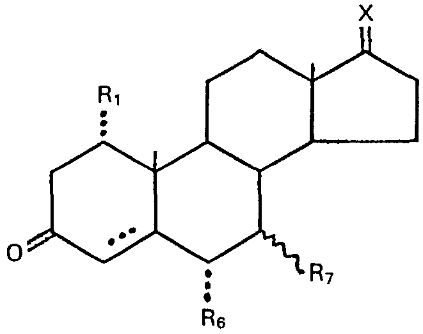

(III),

worin X O, H(OH) oder H(OAc) bedeutet,
wobei Ac für eine niedere 1-4 C-Atome enthaltende Acylgruppe steht,
$R_1$, $R_6$ und $R_7$ die in Formel I angegebene Bedeutung haben und die $\Delta^4$-Doppelbindung fakultativ ist, in 1,2- oder 1,2- und 4,5-Stellung dehydriert und eine freie oder freigesetzte 17-Hydroxygruppe oxydiert oder

c) ein im A-Ring gesättigtes Steroid der allgemeinen Formel IV

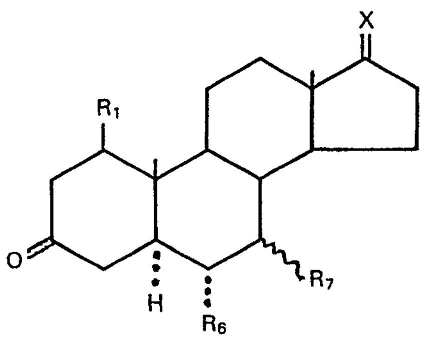

(IV),

worin X O, H(OH) oder H(OAc) bedeutet,
wobei Ac für eine niedere 1-4 C-Atome enthaltende Acylgruppe steht und
$R_1$, $R_6$ und $R_7$ die in Formel I angegebene Bedeutung haben, in 1,2- und 4,5-Stellung dehydriert und eine freie oder freigesetzte 17-Hydroxygruppe oxydiert oder

d) die Alkanoyloxygruppe in einem 1-Alkanoyloxymethylsteroid der allgemeinen Formel I'

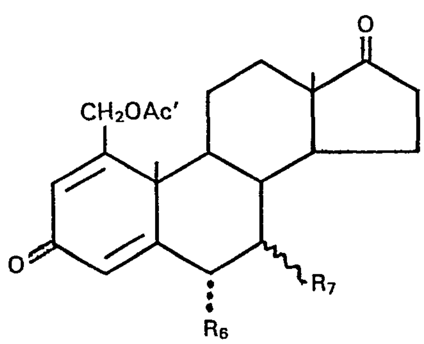

(I'),

worin Ac' eine Alkanoylgruppe mit 1 bis 4 Kohlenstoffatomen darstellt und $R_6$ und $R_7$ die in Formel I angegebene Bedeutung haben, verseift und gegebenenfalls die freigesetzte Hydroxygruppe im 1-Hydroxymethylsteroid in einen $C_{1-3}$-Alkylether oder einen von OAc' verschiedenen $C_{1-4}$-Alkanoylester überführt oder

e) ein Hydroxymethylsteroid der allgemeinen Formel I mit p-Toluolsulfonsäurechlorid und Pyridin zum 1-Tosylmethylsteroid und anschliessend mit Alkalialkoholat zum Alkoxymethylsteroid der allgemeinen Formel I umsetzt.

**Claims for the Designated States:**
BE, CH, DE, FR, GB, IT, LI, LU, NL, SE

1. A 1-alkyl-androsta-1,4-diene-3,17-dione of the general formula I

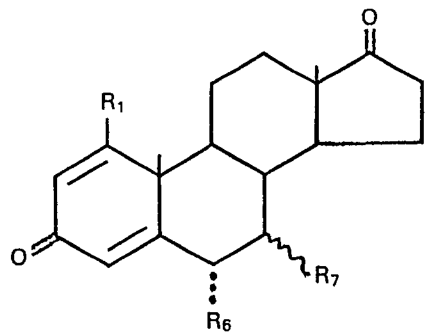

(I),

in which
$R_1$ is a methyl, ethyl, hydroxymethyl, $C_1$-$C_3$-alkoxymethyl or $C_1$-$C_4$-alkanoyloxymethyl group,
$R_6$ is a hydrogen atom or a methyl group, and
$R_7$ is a hydrogen atom or a methyl group in the 7α- or 7β-position.

2. 1-Methylandrosta-1,4-diene-3,17-dione.

3. 1,7α-Dimethylandrosta-1,4-diene-3,17-dione, and 1,7β-Dimethylandrosta-1,4-diene-3,17-dione.

4. 1,6α-Dimethylandrosta-1,4-diene-3,17-dione.

5. 1-Ethylandrosta-1,4-diene-3,17-dione.

6. 1-Acetoxymethylandrosta-1,4-diene-3,17-dione.

7. 1-Hydroxymethylandrosta-1,4-diene-3,17-dione.

8. 1-Ethoxymethylandrosta-1,4-diene-3,17-dione.

9. A pharmaceutical preparation characterised by a content of a compound according to 8 claims.

10. A process for the preparation of a 1-alkyl-androsta-1,4-diene-3,17-dione of the general formula I

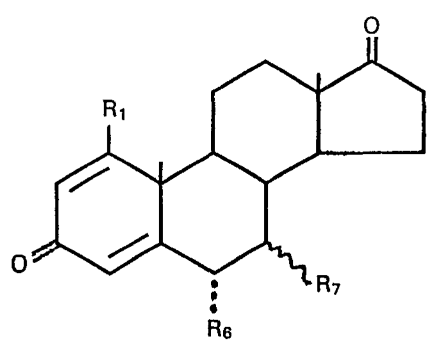

(I),

in which

$R_1$ is a methyl, ethyl, hydroxymethyl, $C_1$-$C_3$-alkoxymethyl or $C_1$-$C_4$-alkanoyloxymethyl group,

$R_6$ is a hydrogen atom or a methyl group, and

$R_7$ is a hydrogen atom or a methyl group in the 7α- or 7β-position

characterised in that

a) the 17-hydroxy group in a 17-hydroxysteroid of the general formula II

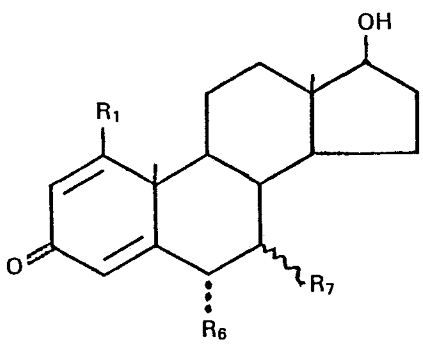

(II),

in which $R_1$, $R_6$ and $R_7$ have the meanings given above, is oxidised to form a 17-oxo group, or

b) a steroid of the general formula III, saturated or singly unsaturated in the A-ring,

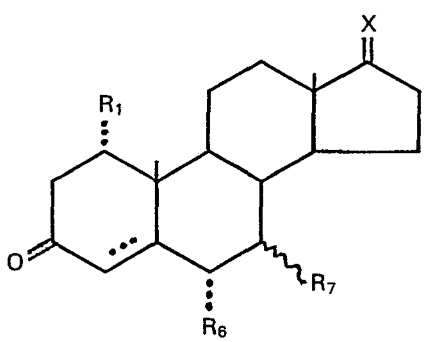

(III),

in which X is O, H(OH) or H(OAc), in which Ac is a lower acyl group containing to 1 to 4 carbon atoms, and $R_1$, $R_6$ and $R_7$ have the meanings given for formula I and the $\Delta^4$ double bond is optional, is dehydrogenated in the 1,2- or 1,2- and 4,5-positions, and any free or liberated 17-hydroxy group is oxidised or

c) a steroid of the general formula IV, saturated in the A ring,

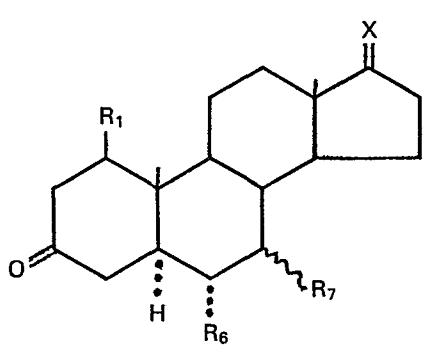

(IV),

in which X is O, H(OH) or H(OAc), in which Ac is a lower acyl group containing 1 to 4 carbon atoms, and $R_1$, $R_6$ and $R_7$ have the meanings given for formula I is dehydrogenated in the 1,2- and 4,5-positions and any free or liberated 17-hydroxy group is oxidised or

d) the alkanoyloxy group in a 1-alkanoyloxymethylsteroid of the general formula I'

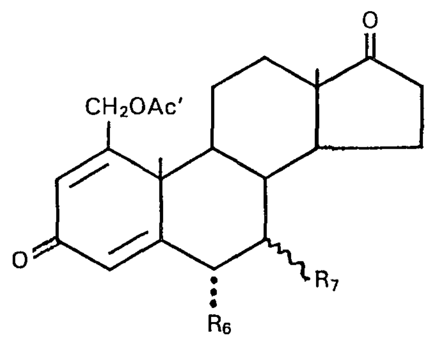

(I'),

in which Ac' is an alkanoyl group containing 1 to 4 carbon atoms, and $R_6$ and $R_7$ have the meanings given in formula I is saponified and, if desired, the liberated hydroxyl group in the 1-hydroxymethylsteroid is converted into a $C_{1\text{-}3}$-alkoxy group or into a $C_{1\text{-}4}$-alkanoyloxy group that is different from OAc' or

e) a 1-hydroxymethylsteroid of general formula I is converted with p-toluenesulphonyl chloride and pyridine to the 1-tosylmethylsteroid and then with an alkali metal alcoholate to the alkoxymethylsteroid of general formula I.

**Claim for the Designed State:** AT

A process for the preparation of a 1-alkyl-androsta-1,4-diene-3,17-dione of the general formula I

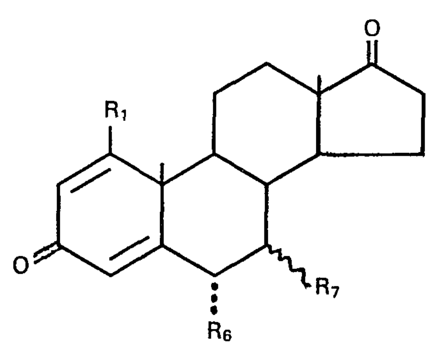

(I),

in which

$R_1$ is a methyl, ethyl, hydroxymethyl, $C_1$-$C_3$-alkoxymethyl or $C_1$-$C_4$-alkanoyloxymethyl group,

$R_6$ is a hydrogen atom or a methyl group, and

$R_7$ is a hydrogen atom or a methyl group in the 7α- or 7β-position

characterised in that

a) the 17-hydroxy group in a 17-hydroxysteroid of the general formula II

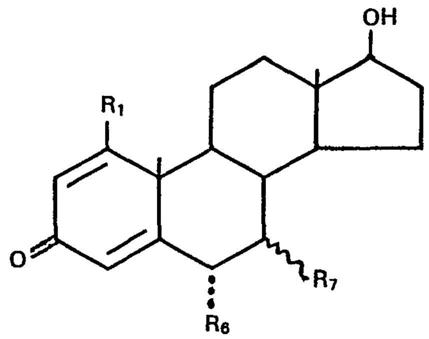

(II),

in which $R_1$, $R_6$ and $R_7$ have the meanings given above, is oxidised to form a 17-oxo group, or

b) a steroid of the general formula III, saturated or singly unsaturated in the A-ring,

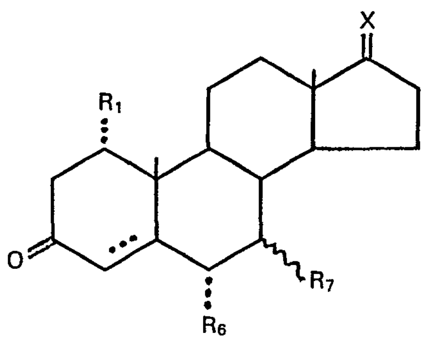

(III),

in which X is O, H(OH) or H(OAc), in which Ac is a lower acyl group containing to 1 to 4 carbon atoms, and $R_1$, $R_6$ and $R_7$ have the meanings given for formula I and the $\Delta^4$ double bond is optional, is dehydrogenated in the 1,2- or 1,2- and 4,5-positions, and any free or liberated 17-hydroxy group is oxidised or

c) a steroid of the general formula IV, saturated in the A ring,

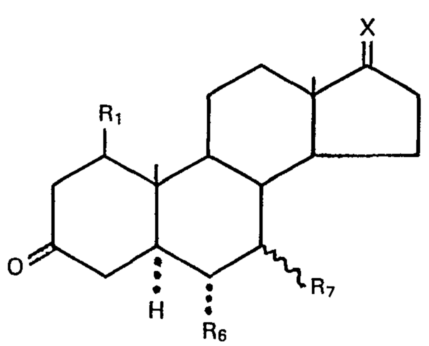

(IV),

in which X is O, H(OH) or H(OAc), in which Ac is a lower acyl group containing 1 to 4 carbon atoms, and $R_1$, $R_6$ and $R_7$ have the meanings given for formula I is dehydrogenated in the 1,2- and 4,5-positions and any free or liberated 17-hydroxy group is oxidised or

d) the alkanoyloxy group in a 1-alkanoyloxymethylsteroid of the general formula I′

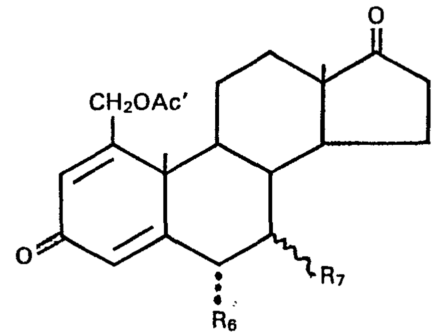

(I′),

in which Ac′ is an alkanoyl group containing 1 to 4 carbon atoms, and $R_6$ and $R_7$ have the meanings given in formula I is saponified and, if desired, the liberated hydroxyl group in the 1-hydroxymethylsteroid is converted into a $C_{1-3}$-alkoxy group or into a $C_{1-4}$-alkanoyloxy group that is different from OAc′ or

e) a 1-hydroxymethylsteroid of general formula I is converted with p-toluenesulphonyl choride and pyridine to the 1-tosylmethylsteroid and then with an alkali metal alcoholate to the alkoxymethylsteroid of general formula I.

**Revendications pour les Etats contractants:**
BE, CH, DE, FR, GB, IT, LI, LU, NL, SE

1. Alkyl-1 androstadiène-1,4 diones-3,17 répondant à la formule générale I

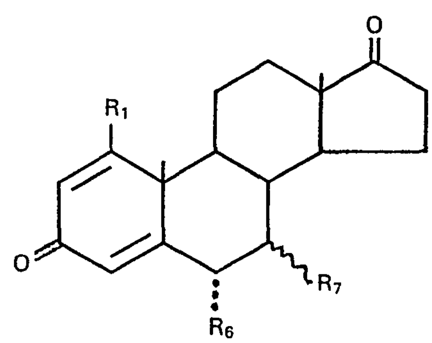

(I),

dans laquelle

$R_1$ représente un radical méthyle, éthyle, hydroxyméthyle, alcoxyméthyle à alcoxy en $C_1$-$C_3$ ou alcanoyloxyméthyle à alcanoyle en $C_1$-$C_4$,

$R_6$ représente un atome d'hydrogène ou un radical méthyle et

$R_7$ représente un atome d'hydrogène ou un radical méthyle en position tα ou 7β.

2. Méthyl-1-androstadiène-1,4 dione-3,17.

3. Diméthyl-1,7α androstadiène-1,4 dione-3,17 et diméthyl-1,7β androstadiène-1,4 dione-3,17.

4. Diméthyl-1,6α androstadiène-1,4 dione-3,17.

5. Ethyl-1 androstadiène-1,4 dione-3,17.

6. Acétoxyméthyl-1 androstadiène-1,4 dione-3,17.

7. Hydroxyméthyl-1 androstadiène-1,4 dione-3,17.

8. Ethoxyméthyl-1 androstadiène-1,4 dione-3,17.

9. Compositions pharmaceutiques caractérisées en ce qu'elles contiennent un composé selon l'une quelconque des revendications 1 à 8.

10. Procédé pour préparer des alkyl-1 androstadiène-1,4 diones-3,17 répondant à la formule générale I

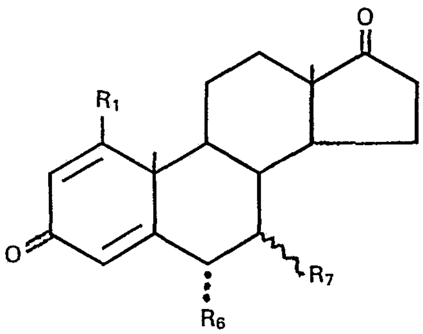

(I),

dans laquelle
$R_1$ représente un radical méthyle, éthyle, hydroxyméthyle, alcoxyméthyle à alcoxy en $C_1$-$C_3$ ou alcanoyloxyméthyle à alcanoyle en $C_1$-$C_4$,
$R_6$ représente un atome d'hydrogène ou un radical méthyle et
$R_7$ représente un atome d'hydrogène ou un radical méthye en position 7α ou 7β,
procédé caractérisé en ce que:

a) dans un hydroxy-17 stéroïde répondant à la formule générale II

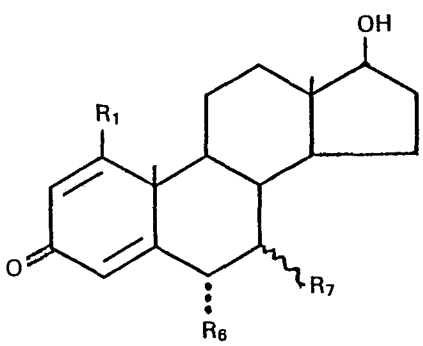

(II),

dans laquelle $R_1$, $R_6$ et $R_7$ ont les significations qui ont été données ci-dessus à propos de la formule I, on oxyde le radical hydroxy en 17 en un radical oxo en 17, ou

b) on déshydrogène un stéroïde dont le noyau A est saturé ou ne contient qu'une seule double liaison et qui répond à la formule générale III

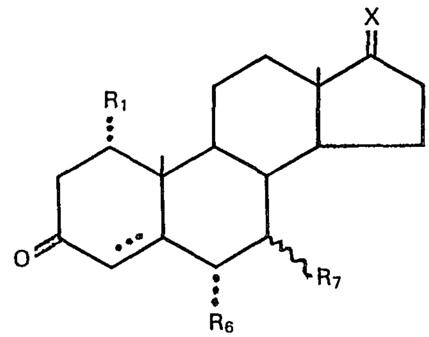

(III),

dans laquelle X représente O, H(OH) ou H(OAc), le symbole Ac représentant un radical acyle inférieur contenant de 1 à 4 atomes de carbone, $R_1$, $R_6$ et $R_7$ ont les significations indiquées ci-dessus à propos de la formule I, et la double liaison $\Delta^4$ est facultative, en position 1,2 ou à la fois en position 1,2 et en position 4,5 et on oxyde un radical hydroxy en 17 libre ou libéré,

c) on déshydrogène un stéroïde à noyau A saturé qui répond à la formule générale IV

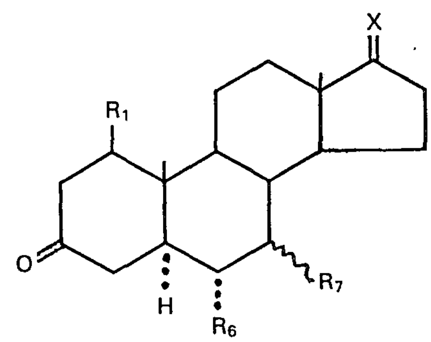

(IV),

dans laquelle X représente O, H(OH) ou H(OAc), le symbole Ac représentant un radical acyle inférieur contenant de 1 à 4 atomes de carbone, et $R_1$, $R_6$ et $R_7$ ont les significations qui leur ont été données à propos de la formule I,
en position 1, 2 et en position 4,5, et on oxyde un radical hydroxy en 17 libre ou libéré ou

d) on saponifie le radical alcanoyloxy d'un alcanoyloxyméthyl-1 stéroïde répondant à la formule générale I'

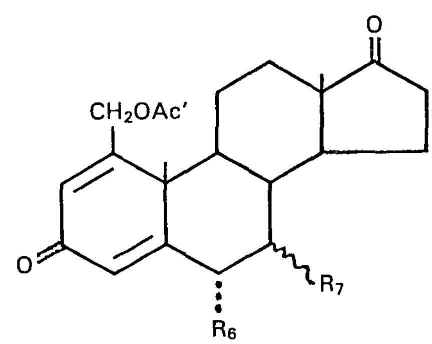

(I'),

dans laquelle Ac' représente un radical alcanoyle contenant de 1 à 4 atomes de carbone tandis que $R_6$ et $R_7$ ont les significations données à propos de la formule I,

et, dans l'hydroxyméthyl-1 stéroïde, on transforme éventuellement le radical hydroxy libéré en un radical alcoxy en $C_1$-$C_3$ ou en un radical alcanoyloxy en $C_1$-$C_4$ différent du radical OAc', ou

e) on fait réagir un hydroxyméthyl-1 stéroïde de formule générale I avec le chlorure de p-toluène-sulfonyle et la pyridine pour le convertir en le tosyloxyméthyl-1 stéroïde, puis on fait reagir ce dernier avec un alcoolate de métal alcalin afin d'obtenir l'alcoxyméthyl-stéroïde de formule générale I.

**Revendications pour l'Etat contractant:** AT

Procédé pour préparer des alkyl-1 androstadiène-1,4 diones-3,17 répondant à la formule générale I

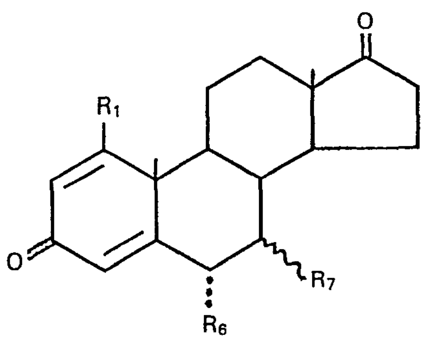

(I),

dans laquelle

$R_1$ représente un radical méthyle, éthyle, hydroxyméthyle, alcoxyméthyle à alcoxy en $C_1$-$C_3$ ou alcanoyloxyméthyle à alcanoyle en $C_1$-$C_4$,

$R_6$ représente un atome d'hydrogène ou un radical méthyle et

$R_7$ représente un atome d'hydrogène ou un radical méthye en position 7α ou 7β,

procédé caractérisé en ce que:

a) dans un hydroxy-17 stéroïde répondant à la formule générale II

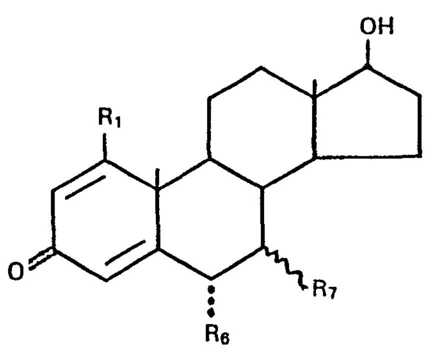

(II),

dans laquelle $R_1$, $R_6$ et $R_7$ ont les significations qui ont été données ci-dessus à propos de la formule I, on oxyde le radical hydroxy en 17 en un radical oxo en 17, ou

b) on déshydrogène un stéroïde dont le noyau A est saturé ou ne contient qu'une seule double liaison et qui répond à la formule générale III

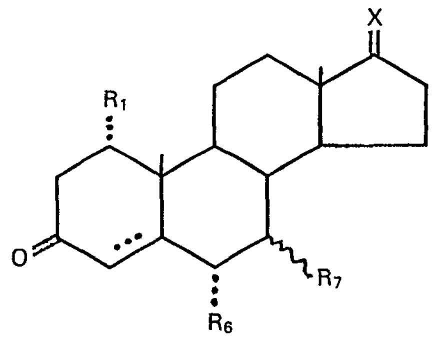

(III),

dans laquelle X représente I, H(OH) ou H(OAc), le symbole Ac représentant un radical acyle inférieur contenant de 1 à 4 atomes de carbone, $R_1$, $R_6$ et $R_7$ ont les significations indiquées au-dessous de la formule I, et la double liaison $\Delta^4$ est facultative, en position 1,2 ou à la fois en position 1,2 et en position 4,5 et on oxyde un radical hydroxy en 17 libre ou libéré,

c) on déshydrogène un stéroïde à noyau A saturé qui répond à la formule générale IV

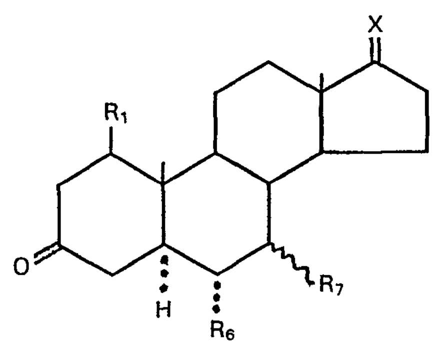

(IV),

dans laquelle X représente O, H(OH) ou H(OAc), le symbole Ac représentant un radical acyle inférieur contenant de 1 à 4 atomes de carbone, et $R_1$, $R_6$ et $R_7$ ont les significations qui leur ont été données à propos de la formule I, en position 1, 2 et en position 4,5, et on oxyde un radical hydroxy en 17 libre ou libéré ou

d) on saponifie le radical alcanoyloxy d'un alcanoyloxyméthyl-1 stéroïde répondant à la formule générale I'

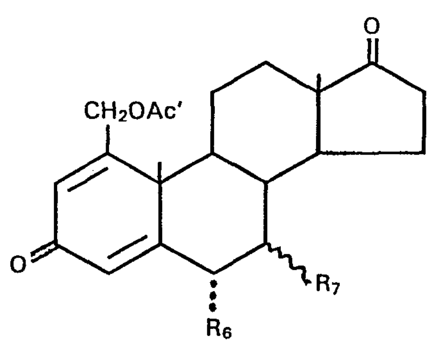

(I'),

dans laquelle Ac' représente un radical alcanoyle contenant de 1 à 4 atomes de carbone tandis que $R_6$

et $R_7$ ont les significations données à propos de la formule I,
et, dans l'hydroxyméthyl-1 stéroïde, on transforme éventuellement le radical hydroxy libéré en un radical alcoxy en $C_1$-$C_3$ ou en un radical alcanoyloxy en $C_1$-$C_4$ différent du radical OAc', ou

e) on fait réagir un hydroxyméthyl-1 stéroïde de formule générale I avec le chlorure de p-toluène-sulfonyle et la pyridine pour le convertir en le tosyloxyméthyl-1 stéroïde, puis on fait reagir ce dernier avec un alcoolate de métal alcalin afin d'obtenir l'alcoxyméthyl-stéroïde de formule générale I.